# EUROPÄISCHE PATENTSCHRIFT

(11) **EP 0 876 824 B1**
(45) Veröffentlichungstag und Bekanntmachung des Hinweises auf die Patenterteilung: **11.12.2002**
(21) Anmeldenummer: 98106115.3
(22) Anmeldetag: 03.04.1998
(51) Int. Cl.: A61M 5/32, A61M 5/50

(54) **Nadelschutz für Glasspritzen mit eingeklebter Nadel**
Needle guard for glass syringes with bonded needles
Protection d'aiguille pour des seringues en verre avec aiguilles collées

(30) Priorität: 09.05.1997 DE 29708314 U
(43) Veröffentlichungstag der Anmeldung: 11.11.1998
(73) Patentinhaber: Bünder Glas GmbH, 32257 Bünde (DE)
(72) Erfinder: Amann, Wolf-Rüdiger, 32429 Minden (DE); Stohlmann, Erhard,, 32257 Bünde (DE)
(74) Vertreter: Schirmer, Siegfried, Dipl.-Ing.

(56) Entgegenhaltungen:
- EP-A- 0 240 787
- WO-A-93/10840
- WO-A-94/22511

## Beschreibung

Die Erfindung betrifft einen Nadelschutz für Glasspritzen mit eingeklebter Nadel.

Für Glasspritzen mit eingeklebter Nadel ist es bekannt, die Nadel mit einem aufschiebbaren Nadelschutzteil aus Gummi zu verschließen. Dieses Nadelschutzteil umschließt aufgrund seiner Elastizität die Nadelspitze und kann problemlos abgezogen werden. Dieses leichte Abziehen des Nadelschutzteils und das mögliche Wiederaufschieben auf die Nadel ist jedoch nicht kontrollierbar, so daß Manipulationen an der Füllung (Serum) der Glasspritze nicht auszuschließen sind. Des weiteren ist es bekannt, die Nadel in einem Kunststoff zu lagern und zu verkleben, wobei das Kunststoffteil zusammen mit der Nadel auf die Glasspritze aufgeschoben wird.

Ferner ist aus der EP 0 240 787 ein Nadelschutz für Glasspritzen mit eingeklebter Nadel bekannt, der einstückig ausgebildet ist und ein offenes vorderes Ende zur Lagerung eines Gummistopfens aufweist, wobei auf einer inneren Wandung zur Lagerung des Gummistopfens eine untere und eine obere Halterung angeordnet sind. Bei dem bekannten Nadelschutz wird die obere Halterung dadurch gebildet, daß die Wandung nach Einschieben des Gummistopfens nach innen verformt wird, was insofern nachteilig ist, als ein zusätzlicher Arbeitsschritt erforderlich ist.

Ein Nadelschutz für Glasspritzen mit eingeklebter Nadel, der mit einer Sollbruchstelle ausgebildet ist, ist aus der WO 9422511A bekannt.

Der Erfindung liegt die Aufgabe zugrunde, einen Nadelschutz für Glasspritzen mit eingeklebter Nadel zu schaffen, bei dem bei Gewährleistung einer völligen Abdichtung der Nadelspitze eine nachträgliche Manipulation an der Spritzenfüllung sofort erkennbar ist und der insbesondere kostengünstig herzustellen und einfach zu montieren ist.

Diese Aufgabe wird erfindungsgemäß durch einen Nadelschutz nach Anspruch 1 gelöst.

In Ausgestaltung der Erfindung ist im unteren Bereich eine nach innen weisende Ringnut zur Verrastung mit dem Konus der Glasspritze angeordnet. Die darüberliegende Sollbruchstelle kann umlaufend angeordnet sein. Zweckmäßigerweise ist die Sollbruchstelle durch eine Querschnittsschwächung gebildet. Nach einem weiteren Merkmal der Erfindung befindet sich die Sollbruchstelle im aufgebrachten Zustand des Nadelschutzes oberhalb oder auf der Höhe der oberen Begrenzung der Glasspritze. Zur Lagerung des Gummistopfens kann auf der inneren Wandung des Nadelschutzes eine untere und eine obere Halterung (Stege) angeordnet sein, wobei der lichte Abstand zwischen den Halterungen kleiner oder etwa gleich ist als die Höhe des Gummistopfens vor dem Einführen in den Nadelschutz. Vorteilhafterweise besitzt die obere Halterung eine Auflauffläche. Die Halterungen sind zweckmäßigerweise durch je eine umlaufende Ringwulst oder in Form von Stegen gebildet. Der Nadelschutz ist bei einer bevorzugten Ausführung aus elastischem Material hergestellt. Er kann im Spritzgießverfahren aus Chemiewerkstoff hergestellt sein.

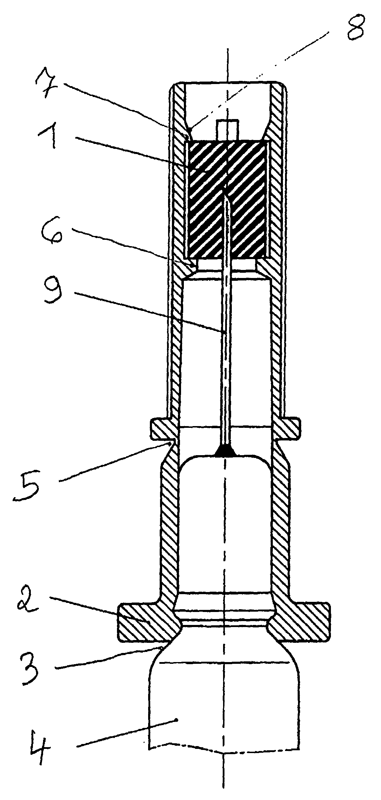

Ein Ausführungsbeispiel der Erfindung ist in der Zeichnung dargestellt und wird nachfolgend näher beschrieben. Die einzige Figur zeigt einen Vertikalschnitt durch einen auf eine Glasspritze aufgeschobenen Nadelschutz.

Der Nadelschutz weist im unteren Bereich eine nach innen weisende Ringwulst 2 auf, die in den einen entsprechend ausgebildeten Einrastring aufweisenden Konus 3 der Glasspritze 4 verrastbar ist. Da der Nadelschutz aus einem elastischen Material, vorzugsweise Kunststoff, besteht, ist das Verrasten relativ leicht. Dadurch ist ein fester Sitz des Nadelschutzes auf der Glasspritze 4 gegeben. Eine vorgesehene Sollbruchstelle 5, die durch eine umlaufende Querschnittsschwächung gebildet ist und die sich im aufgebrachten Zustand des Nadelschutzes oberhalb oder in gleicher Höhe der oberen Begrenzung der Glasspritze 4 befindet, ermöglicht ein Abdrehen des oberen Bereichs des Nadelschutzes und damit ein Freilegen der Nadel 9.

Zur Abdichtung der Spitze der Nadel 9 dient bekannterweise ein Gummistopfen 1. Dieser Gummistopfen 1 wird von der oberen freien Stirnfläche aus in den Nadelschutz eingedrückt und ist darin sicher gehaltert. Hierzu dienen auf der inneren Wandung des Nadelschutzes vorhandene untere 6 und obere Halterungen 7, deren lichter Abstand etwa gleich ist wie die Höhe des Gummistopfens 1 vor dem Einführen in den Nadelschutz. Zur Erleichterung des Einführens des Gummistopfens 1 weist die obere Halterung 7 eine Auflauffläche 8 auf. In Funktionsstellung weist der Gummistopfen 1 eine Vorspannung auf, so daß ein exakter Sitz und damit auch eine exakte Abdichtung der Nadelspitze gewährleistet ist.

## Patentansprüche

1. Nadelschutz für Glasspritzen mit eingeklebter Nadel, der einstückig mit einer Sollbruchstelle (5) ausgebildet ist und ein offenes vorderes Ende zur Lagerung eines Gummistopfens (1) aufweist, wobei auf einer inneren Wandung zur Lagerung des Gummistopfens (1) eine untere (6) und eine obere Halterung (7) angeordnet sind, **dadurch gekennzeichnet, daß** im montierten Zustand des Gummistopfens das vordere Ende des Nadelschutzes eine freie Stirnfläche aufweist, durch die der Gummistopfen (1) eindrückbar ist.

2. Nadelschutz nach Anspruch 1, **dadurch gekennzeichnet, daß** im unteren Bereich eine nach innen weisende Ringwulst (2) zur Verrastung mit dem Konus (3) der Glasspritze (4) angeordnet ist.

3. Nadelschutz nach Anspruch 1 oder 2, **dadurch gekennzeichnet, daß** die Sollbruchstelle (5) umlaufend angeordnet ist.

4. Nadelschutz nach einem der Ansprüche 1 bis 3, **dadurch gekennzeichnet, daß** die Sollbruchstelle (5) durch eine Querschnittsschwächung gebildet ist.

5. Nadelschutz nach einem der Ansprüche 1 bis 4, **dadurch gekennzeichnet, daß** sich die Sollbruchstelle (5) im aufgebrachten Zustand des Nadelschutzes oberhalb oder in gleicher Höhe der oberen Begrenzung der Glasspritze (4) befindet.

6. Nadelschutz nach Anspruch 5, **dadurch gekennzeichnet, daß** der lichte Abstand zwischen den Halterungen (6; 7) kleiner oder etwa gleich ist als die Höhe des Gummistopfens (1) vor dem Einführen in den Nadelschutz.

7. Nadelschutz nach Anspruch 5 oder 6, **dadurch gekennzeichnet, daß** die obere Halterung (7) eine Auflauffläche (8) aufweist.

8. Nadelschutz nach einem der Ansprüche 5 bis 7, **dadurch gekennzeichnet, daß** die Halterungen (6; 7) durch je eine umlaufende Ringwulst oder in Form von Stegen gebildet sind.

9. Nadelschutz nach einem der Ansprüche 1 bis 8, **dadurch gekennzeichnet, daß** er aus elastischem Material gebildet ist.

10. Nadelschutz nach Anspruch 9, **gekennzeichnet durch** die Herstellung im Spritzgießverfahren aus Chemiewerkstoff.

## Claims

1. Needle guard for glass syringes with bonded needle, the guard being integrally formed with a frangible location (5) and having an open front end for mounting of a rubber plug (1), wherein a lower retainer (6) and an upper retainer (7) for retaining the rubber plug (1) are arranged on an inner wall, **characterised in that** in the mounted state of the rubber plug the front end of the needle guard has a free end face through which the rubber plug (1) can be pressed in.

2. Needle guard according to claim 1, **characterised in that** an inwardly facing annular bead (2) for detenting with the cone (3) of the glass syringe (4) is arranged in the lower region.

3. Needle guard according to claim 1 or 2, **characterised in that** the frangible location (5) is arranged to be encircling.

4. Needle guard according to one of claims 1 to 3, **characterised in that** the frangible location (5) is formed by a cross-sectional weakening.

5. Needle guard according to one of claims 1 to 4, **characterised in that** the frangible location (5) in the mounted state of the needle guard is disposed above or at the same height as the upper boundary of the glass syringe (4).

6. Needle guard according to claim 5, **characterised in that** the clear spacing between the retainers (6; 7) is smaller than or approximately equal to the height of the rubber plug (1) before introduction into the needle guard.

7. Needle guard according to claim 5 or 6, **characterised in that** the upper retainer (7) has an entry surface (8).

8. Needle guard according to one of claims 5 to 7, **characterised in that** the retainers (6; 7) are each formed by a respective encircling annular bead or are formed in the form of webs.

9. Needle guard according to one of claims 1 to 8, **characterised in that** it is formed from resilient material.

10. Needle guard according to claim 9, **characterised by** manufacture in an injection moulding process from a chemical material.

## Revendications

1. Protection d'aiguille pour des seringues en verre à aiguille collée, réalisée en une seule pièce avec un point de rupture de consigne (5) et une extrémité avant ouverte pour un bouchon en caoutchouc (1), la paroi intérieure étant munie d'un moyen de fixation inférieur (6) et d'un moyen de fixation supérieur (7) pour tenir le bouchon en caoutchouc (1),
**caractérisée en ce qu'**
à l'état monté du bouchon en caoutchouc, l'extrémité avant de la protection d'aiguille présente une surface frontale libre qui peut être enfoncée par le bouchon en caoutchouc (1).

2. Protection d'aiguille selon la revendication 1,
**caractérisée en ce que**
dans la zone inférieure, un bourrelet annulaire (2) tourné vers l'intérieur s'accroche au cône (3) de la seringue en verre (4).

3. Protection d'aiguille selon l'une quelconque des revendications 1 ou 2,
**caractérisée en ce que**
le point de rupture de consigne (5) est périphérique.

4. Protection d'aiguille selon l'une quelconque des revendications 1 à 3,
**caractérisée en ce que**
le point de rupture de consigne (5) est formé par un affaiblissement de la section.

5. Protection d'aiguille selon l'une quelconque des revendications 1 à 4,
**caractérisée en ce que**
le point de rupture de consigne (5), à l'état installé de la protection d'aiguille, se trouve au-dessus ou à même hauteur que la limite supérieure de la seringue en verre (4).

6. Protection d'aiguille selon la revendication 5,
**caractérisée en ce que**
la distance libre entre les moyens de fixation (6, 7) est inférieure ou sensiblement égale à la hauteur du bouchon en caoutchouc (1) avant son introduction dans la protection d'aiguille.

7. Protection d'aiguille selon l'une quelconque des revendications 5 ou 6,
**caractérisée en ce que**
la fixation supérieure (5) comporte une surface d'attaque (8),

8. Protection d'aiguille selon l'une quelconque des revendications 5 à 7,
**caractérisée en ce que**
les fixations (6, 7) sont formées chacune par un bourrelet annulaire périphérique ou des entretoises.

9. Protection d'aiguille selon l'une quelconque des revendications 1 à 8,
**caractérisée en ce qu'**
elle est réalisée en matière élastique.

10. Protection d'aiguille selon la revendication 9,
**caractérisée par**
la fabrication selon un procédé d'injection d'une matière chimique.
